(19) **Europäisches Patentamt / European Patent Office / Office européen des brevets**

(11) **EP 2 044 946 B1**

(12) **EUROPEAN PATENT SPECIFICATION**

(45) Date of publication and mention
of the grant of the patent:
**09.02.2011 Bulletin 2011/06**

(51) Int Cl.:
*A61K 33/26* (2006.01)     *A61P 7/08* (2006.01)
*A61P 13/12* (2006.01)     *B01J 20/06* (2006.01)

(21) Application number: **06767582.7**

(22) Date of filing: **29.06.2006**

(86) International application number:
**PCT/JP2006/312965**

(87) International publication number:
**WO 2008/001443 (03.01.2008 Gazette 2008/01)**

(54) **PREVENTIVE REMEDIAL THERAPEUTIC AGENT FOR PHOSPHORUS IMPAIRMENT, ORAL AGENT FOR ADSORBING PHOSPHATE ION CONTAINED IN FOOD, BEVERAGE AND CHEMICAL, AND PROCESS FOR PRODUCING THEM**

PRÄVENTIVES UND THERAPEUTISCHES MITTEL GEGEN PHOSPHOR-STÖRUNG, ORALES MITTEL ZUR ADSORPTION VON PHOSPHATIONEN IN NAHRUNGSMITTELN, GETRÄNKEN UND CHEMIKALIEN UND HERSTELLUNGSVERFAHREN

AGENT THÉRAPEUTIQUE PRÉVENTIF POUR DÉFICIENCE EN PHOSPHORE, AGENT ORAL POUR ADSORPTION D'IONS DE PHOSPHATE CONTENUS DANS UN ALIMENT, UNE BOISSON ET UNE SUBSTANCE CHIMIQUE ET LEUR PROCÉDÉ DE PRÉPARATION

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**08.04.2009 Bulletin 2009/15**

(73) Proprietor: **J-Pharma Co., Ltd.**
**Tokyo 160-0022 (JP)**

(72) Inventors:
• **ENDOU, Hitoshi**
**Tokyo; 160-0022 (JP)**
• **YANAGITA, Tomotaka**
**Tokyo; 156-0052 (JP)**
• **YAMASHITA, Koji**
**Tokyo; 171-0041 (JP)**

(74) Representative: **Brochard, Pascale**
**Osha Liang**
**32 avenue de l'Opéra**
**75002 Paris (FR)**

(56) References cited:
**WO-A1-03/053565        GB-A- 748 024**
**JP-A- 05 155 776        JP-A- 2000 506 372**

JP-A- 2001 517 633        US-A1- 2004 186 073

• SAVICA VINCENZO ET AL: "Phosphate binders and management of hyperphosphataemia in end-stage renal disease." NEPHROLOGY, DIALYSIS, TRANSPLANTATION : OFFICIAL PUBLICATION OF THE EUROPEAN DIALYSIS AND TRANSPLANT ASSOCIATION - EUROPEAN RENAL ASSOCIATION AUG 2006, vol. 21, no. 8, August 2006 (2006-08), pages 2065-2068, XP002546960 ISSN: 0931-0509
• YAMAGUCHI T ET AL: "Oral phosphate binders: phosphate binding capacity of iron (III) hydroxide complexes containing saccharides and their effect on the urinary excretion of calcium and phosphate in rats." RENAL FAILURE SEP 1999, vol. 21, no. 5, September 1999 (1999-09), pages 453-468, XP009123066 ISSN: 0886-022X
• YAMAGUCHI T. ET AL.: 'Glucosamine Tenka ni yoru Muteikei Suisankatetsu no Gosei to Keiko Phosphorous Kyuchakuzai to shite no Hyoka (Synthesis of Amorphous Iron Hydroxide in the Presence of Glucosamine and its Evaluation as Phosphate Adsorption Agent for Oral Administration)' DAI 72 KAI CSJ: THE CHEMICAL SOCIETY OF JAPAN KOEN YOKOSHU 1997, page 1271 + ABSTR. NO. 4K107, XP003019894

EP 2 044 946 B1

• **IDA S. ET AL.: 'Keiko Phosphorous Kyuchakuzai to shite no Hishoshitsu Suisankatetsu (III)' JOURNAL OF THE CHEMICAL SOCIETY OF JAPAN no. 1, 1995, pages 19 - 24, XP003019895**

**Description**

TECHNICAL FIELD

The present invention relates to agents and oral preparations capable of efficiently adsorbing phosphate ions for which excessive intake causes problems. More specifically, the present invention relates to (1) agents for preventing, improving or treating organ disorders due to phosphorus, which are capable of improving blood concentrations of phosphorus of patients with kidney failures and controlling accumulations of phosphorus in the body and (2) oral preparations for preventing various diseases related to phosphorus, which are capable of eliminating problems with excessive intake of phosphorus from foods and beverages or the like.

BACKGROUND ART

Phosphorus is an essential substance for living organisms. Man decomposes foods into phosphate ions throughout the digestive tract (small intestine, in particular) and subsequently absorbed. The average Japanese adult has a daily phosphorus intake of approximately 1,000 mg. It is reported that, for a normal person, approximately 80% of such phosphorus is absorbed and approximately 80% of that absorbed portion is excreted by the kidney. If the kidney dysfunctions (renal failure), phosphorus excretion decreases and causes an increased serum phosphorus concentration (a hyperphosphatemia). A healthy human maintains a phosphorus serum concentration range of 0.25 to 4.5 mg/dl; concentrations at or over 4.5 mg/dl are defined as hyperphosphatemia.

Hyperphosphatemia induces abnormal calcium metabolism and/or hyperparathyroidism and causes modifications of the bones throughout the body (renal osteodystrophy) and/or calcium deposits formed in various organs - especially to the cardiac valves, aortae, lungs and the like (heterotopic calcinosis). Such disorders not only impair the QoL (quality of life) of patients with renal failures but also cause fatal complications such as cardiac infarction and exacerbating life prognoses. Furthermore, hyperphosphatemia is known to be a promoting factor of renal disorders. Thus, the ability to maintain a normal (healthy) phosphorus blood concentration is a crucial problem for patients with renal failures.

As described above, the absolute amount of phosphorus that the kidney can excrete will decrease under renal failure. Hence, under renal failure, when the amount of phosphorus absorbed via the digestive tract exceeds the capacity of the kidney, phosphorus will accumulate in the body. As such, the standard treatment for patients with kidney failures has been to limit the total amount of phosphorus absorbed through the digestive tract; an amount that may not exceed the capacity of the kidney.

In addition to alimentary therapy through phosphorus restricting diet, phosphorus adsorbents (agents capable of reducing the amount of absorbed phosphorus by adsorbing phosphate ions generated from foods in the digestive tract, especially in the small intestine and excreting them directly with stool) have been used in combination for renal failure treatment (Patent References 1 to 4, for example).

However, phosphorus adsorbents, which have been put into practical use or which are under review for clinical applications, suffer from such disadvantages as described below.

Firstly, aluminum preparations (aluminum hydroxide gels) are highly adsorptive to phosphate ions in the digestive tract and reduce phosphorus serum concentration. However, aluminum - once absorbed through the digestive tract - will not be excreted outside the body and can cause aluminum poisoning due to the accumulation of aluminum in the body (aluminum encephalopathy, aluminum osteopathy and anemia). For this reason, administration of the aluminum preparations has been contraindicated for dialysis patients in our nation (Japan) since June, 1992.

Secondly, calcium preparations (such as calcium carbonate and calcium acetate) are still widely used in place of aluminum preparations. However, they too have disadvantages such as they require large doses to improve hyperphosphatemia and they are usually regarded as distasteful and hard to be taken; furthermore, calcium is in turn absorbed through the digestive tract and can cause hypercalcemia which may bring about additional exacerbations such as heterotopic calcification.

Thirdly, novel substances have been introduced in recent years as phosphorus adsorbents and are now under review for use. One of them, known as Sevelamer HCl™, is a polymer of prop-2-en-1-amine and 1-chloro-2,3-epoxypropane in the hydrochloride form. Sevelamer HCl™ is a phosphorus binding polymer developed in the United States, and approved in Japan in January, 2003 and currently used. However, this agent often needs to be administered in large doses in order to improve hyperphosphatemia. In addition, there are still a number of problems yet to be solved such as frequent complications with the digestive tract(i.e. constipation). Another reagent, lanthanum carbonate, is under review for use in the United States and Europe. Since the influences of lanthanum on living organisms are not yet sufficiently understood, which may have similar problems as aluminum preparations, its practical application appears very questionable.

EP 2 044 946 B1

Patent Reference 1: Japanese Unexamined Patent Publication No. 1990-77266
Patent Reference 2: Japanese Unexamined Patent Publication No. 1991-182259
Patent Reference 3: Japanese Unexamined Patent Publication No. 1995-2903
Patent Reference 4: PCT Publication WO 01/66607

DISCLOSURE OF THE INVENTION

PROBLEMS TO BE SOLVED BY THE INVENTION

Further, since 1999, various iron compounds (stabilized polynuclear iron hydroxide, iron(3)-saccharide complex, iron(3)-sucrose complex, ferric polymaltose complex, ferric citrate) have been under review for use as phosphorus adsorbents. However, these iron compounds suffer from the disadvantages that they are insufficient in phosphorus adsorptive power and need to be administered in large doses in order to improve hyperphosphatemia, as in the case of calcium carbonate and Sevelamer HCl™. These iron substances do not accumulate in the body (iron poisoning) differently from aluminum gel and do not create serious digestive tract complications differently from Sevelamer HCl, when clinically applied. As such, focusing attention on iron compounds having such advantages, the first object of the present invention is to provide agents for preventing, improving or treating phosphorus-related disorders; agents which are biostable and more potent, by enhancing phosphorus adsorptive power of the iron compounds to the degrees equal to or higher than that of conventional adsorbents.

In addition, when phosphorus-containing foods, beverages are taken in large quantities or phosphate-containing agents are taken, high phosphate ions will emerge through the digestive tract. The large quantities of phosphoric acid produced in the digestive tract will then be absorbed in large quantities through the mucosa of the digestive tract and, when the excretion capacity of the kidney becomes exceeded, phosphorus can start accumulating in the body; a condition resulting in the phosphorus-related disorders previously described above. As such, it is a second object of the present invention to provide oral preparations (food and beverage additives, food and beverage adjuvants, pharmaceutical additives or pharmaceutical adjuvants, for example) having as an active principle an iron compound with high adsorptive power to phosphate ions that are effective in preventing various diseases directly or indirectly related to phosphate ions by adsorbing phosphate ions produced by digestion and decomposition of foods and beverages or pharmaceuticals.

MEANS FOR SOLVING THE PROBLEMS

The present invention is the agent for preventing, improving or treating a phosphorus-related disorder according to the invention wherein the ferric hydroxide is produced by adding to an aqueous solution of ferrous iron an oxidizing agent in an amount less than the equivalent amount of the ferrous iron and then adding an alkali in such a manner that its pH at the end of the reaction may be adjusted in the range of 1.5 to 5.5 (preferably 1.5 to 4.0 and more preferably 2.0 to 3.5).

The present invention is the agent for preventing, improving or treating a phosphorus-related disorder according to the invention wherein the ferric hydroxide is produced by adding an oxidizing agent to an aqueous solution of ferrous iron in such a manner that its redox potential may be brought in the range of +400 to 770 mV (preferably +500 to 730 mV and more preferably +600 to 700 mV) and then adding an alkali in such a manner that its pH at the end of the reaction may be adjusted in the range of 1.5 to 5.5 (preferably 1.5 to 4.0 and more preferably 2.0 to 3.5).

The present invention is the agent for preventing, improving or treating a phosphorus-related disorder according to the invention wherein the ferric hydroxide is produced by adding to an aqueous solution of ferrous iron an oxidizing agent in an amount less than the equivalent amount of the ferrous iron in such a manner that its redox potential may be brought in the range of +400 to 770 mV and then adding an alkali in such a manner that its pH at the end of the reaction may be adjusted in the range of 1.5 to 5.5.

The present invention is the agent for preventing, improving or treating a phosphorus-related disorder according to any one of the inventions wherein the oxidizing agent is a hypochlorite.

The present invention is the agent for preventing, improving or treating a phosphorus-related disorder according to any one of the inventions wherein the ferric hydroxide is amorphous.

The present invention is the agent for preventing, improving or treating a phosphorus-related disorder according to any one of the inventions further including glycerin.

The present inventions is the agent for preventing, improving or treating a phosphorus-related disorder according to any one of the inventions (1) to (7) wherein the disorder is hyperphosphatemia.

The present invention is a process for producing an agent for preventing, improving or treating a phosphorus-related disorder, the agent containing ferric hydroxide, which comprises the steps of adding to an aqueous solution of ferrous iron an oxidizing agent in an amount less than the equivalent amount of the ferrous iron and then adding an alkali in such a manner that its pH may be adjusted in the range of 1.5 to 5.5 (preferably 1.5 to 4.0 and more preferably

4

2.0 to 3.5).

The present invention is a process for producing an agent for preventing, improving or treating a phosphorus-related disorder, the agent containing ferric hydroxide, which comprises the steps of adding an oxidizing agent to an aqueous solution of ferrous iron in such a manner that its redox potential may be brought in the range of +400 to 770 mV (preferably +500 to 730 mV and more preferably +600 to 700 mV) and then adding an alkali in such a manner that its pH may be adjusted in the range of 1.5 to 5.5 (preferably 1.5 to 4.0 and more preferably 2.0 to 3.5).

The present invention is a process for producing an agent for preventing, improving or treating a phosphorus-related disorder, the agent containing ferric hydroxide, which comprises the steps of adding to an aqueous solution of ferrous iron an oxidizing agent in an amount less than the equivalent amount of the ferrous iron in such a manner that its redox potential may be brought in the range of +400 to 770 mV (preferably +500 to 730 mV and more preferably +600 to 700 mV) and then adding an alkali in such a manner that its pH may be adjusted in the range of 1.5 to 5.5 (preferably 1.5 to 4.0 and more preferably 2.0 to 3.5).

The present invention is the process according to any one of the inventions wherein the oxidizing agent is a hypochlorite.

The present invention is the process according to any one of the inventions wherein the ferric hydroxide is amorphous.

The present invention is the process according to any one of the inventions further including the step of adding glycerin.

The present invention is the process according to any one of the inventions further including the step of dehydration, freeze-drying or spray-drying.

The present invention is the process according to the invention wherein, after the step for pH adjustment, the step of adding glycerin is performed before, during or after the step of dehydration, freeze-drying or spray-drying.

The present invention is the process according to any one of the inventions wherein the disorder is hyperphosphatemia.

The present invention is an oral preparation for adsorbing phosphate ions in foods and beverages or pharmaceuticals, which contains ferric hydroxide as produced under such conditions that a ferrous species is present.

The present invention is the oral preparation according to the invention wherein the ferric hydroxide is produced by adding to an aqueous solution of ferrous iron an oxidizing agent in an amount less than the equivalent amount of the ferrous iron and then adding an alkali in such a manner that its pH at the end of the reaction may be adjusted in the range of 1.5 to 5.5 (preferably 1.5 to 4.0 and more preferably 2.0 to 3.5).

The present invention is the oral preparation according to the invention wherein the ferric hydroxide is produced by adding an oxidizing agent to an aqueous solution of ferrous iron in such a manner that its redox potential may be brought in the range of +400 to 770 mV (preferably +500 to 730 mV and more preferably +600 to 700 mV) and then adding an alkali in such a manner that its pH at the end of the reaction may be adjusted in the range of 1.5 to 5.5 (preferably 1.5 to 4.0 and more preferably 2.0 to 3.5).

The present invention is the oral preparation according to the invention wherein the ferric hydroxide is produced by adding to an aqueous solution of ferrous iron an oxidizing agent in an amount less than the equivalent amount of the ferrous iron in such a manner that its redox potential may be brought in the range of +400 to 770 mV (preferably +500 to 730 mV and more preferably +600 to 700 mV) and then adding an alkali in such a manner that its pH at the end of the reaction may be adjusted in the range of 1.5 to 5.5 (preferably 1.5 to 4.0 and more preferably 2.0 to 3.5).

The present invention is the oral preparation according to any one of the inventions wherein the oxidizing agent is a hypochlorite.

The present invention is the oral preparation according to any one of the inventions wherein the ferric hydroxide is amorphous.

The present invention is the oral preparation according to any one of the inventions further including glycerin.

The present invention is the oral preparation according to any one of the inventions which is a food and beverage additive, food and beverage adjuvant, pharmaceutical additive or pharmaceutical adjuvant.

The present invention is a process for producing an oral preparation for adsorbing phosphate ions in foods and beverages or pharmaceuticals, the preparation containing ferric hydroxide, which comprises the steps of adding to an aqueous solution of ferrous iron an oxidizing agent in an amount less than the equivalent amount of the ferrous iron and then adding an alkali in such a manner that its pH may be adjusted in the range of 1.5 to 5.5 (preferably 1.5 to 4.0 and more preferably 2.0 to 3.5).

The present invention is a process for producing an oral preparation for adsorbing phosphate ions in foods and beverages or pharmaceuticals, the preparation containing ferric hydroxide, which comprises the steps of adding an oxidizing agent to an aqueous solution of ferrous iron in such a manner that its redox potential may be brought in the range of +400 to 770 mV (preferably +500 to 730 mV and more preferably +600 to 700 mV) and then adding an alkali in such a manner that its pH may be adjusted in the range of 1.5 to 5.5 (preferably 1.5 to 4.0 and more preferably 2.0 to 3.5).

The present invention is a process for producing an oral preparation for adsorbing phosphate ions in foods and beverages or pharmaceuticals, which comprises the steps of adding to an aqueous solution of ferrous iron an oxidizing agent in an amount less than the equivalent amount of the ferrous iron in such a manner that its redox potential may be brought in the range of +400 to 770 mV (preferably +500 to 730 mV and more preferably +600 to 700 mV) and then adding an alkali in such a manner that its pH may be adjusted in the range of 1.5 to 5.5 (preferably 1.5 to 4.0 and more preferably 2.0 to 3.5).

The present invention is the process according to any one of the inventions wherein the oxidizing agent is a hypochlorite.

The present invention is the process according to any one of the inventions wherein the ferric hydroxide is amorphous.

The present invention is the process according to any one of the inventions further including the step of adding glycerin.

The present invention is the process according to any one of the inventions further including the step of dehydration, freeze-drying or spray-drying.

The present invention is the process according to the invention wherein, after the step for pH adjustment, the step of adding glycerin is performed before, during or after the step of dehydration, freeze-drying or spray-drying.

The present invention is the process according to any one of the inventions wherein the oral preparation is a food and beverage additive, food and beverage adjuvant, pharmaceutical additive or pharmaceutical adjuvant.

Terms as used herein will now be defined. The term "ferrous species" refers to a substance in which iron is present as having a valence of two, such as a ferrous ion or ferrous compound (ferrous hydroxide, for example). The term "aqueous solution of ferrous iron" is not particularly limited as long as it is an aqueous solution in which ferrous ions are present and may contain other substances. The term "oxidizing agent" is not particularly limited, examples of which may include hypochlorites, hydrogen peroxide and calcium hydroperoxide, hypochlorites being preferred. The term "phosphorus-related disorder" refers to a disorder affecting various organs due to an excessive accumulation of phosphorus in the body, often attributable to chronic renal failures. Examples of such diseases and symptoms may include osteopathy, calcium deposits to the organs, such as the heart, aortae and lungs, anemia and secondary hyperparathyroidism. The term "agent for preventing, improving or treating a phosphorus-related disorder" refers to an agent to be used for the purpose of at least one of prevention, improvement and treatment. The term "oral preparation" is not particularly limited as along as it is orally administered, including one to be added to foods or beverages (food and beverage additive), one to be taken separately from foods or beverages (food and beverage adjuvant such as supplement), one to be added to pharmaceuticals (pharmaceutical additive) and one to be taken separately from pharmaceuticals (pharmaceutical adjuvant).

BEST MODE FOR CARRYING OUT THE INVENTION

A best mode of the present invention will be described below. The agent for preventing, improving or treating a phosphorus-related disorder and the oral preparation for adsorbing phosphate ions in foods and beverages or pharmaceuticals differ partly in terms of use (sharing a common purpose of preventing phosphorus-related disorders) but share a common ingredient (phosphate adsorbent). Therefore, description will be made first on the phosphorus adsorbent and then on the respective uses in detail.

The present phosphorus adsorbent includes amorphous ferric hydroxide as produced under such conditions that a ferrous species (ferrous hydroxide, for example) is present. Although the active ingredient exhibiting a high phosphorus adsorptive power is amorphous ferric hydroxide, not any ferric hydroxide can achieve such an effect. For example, ferric hydroxide produced by adding caustic soda to a solution of ferric iron or commercially available ferric hydroxide will not exhibit such a high phosphorus adsorptive power (see Examples). On the basis of Eh (redox potential) vs pH charts for $Fe^{2+}$-$Fe(OH)_3$ systems, the present ferric hydroxide is produced under extremely unstable conditions where iron ions are present as ferric ions while remaining under Eh vs pH conditions where they remain as ferrous iron when they are present as stable chemical species. The present ferric hydroxide therefore includes ferrous iron in ferric hydroxide in the produced precipitate and is in an unstable and extremely high amorphous state. The present ferric hydroxide is therefore characterized in that the bond - Fe-O-Fe-O-Fe- is unstable and easily cleaved and it is presumed that newly produced Fe-OH groups react with phosphate ions and the like while bonds are cleaved, exhibiting remarkably high adsorptive power.

The chemical structure of ferric hydroxide is unclear. On the basis of experimentation results and the like, however, it has presumably the structure as described below (the ferric hydroxide according to the present invention is not, however, limited to such a presumed formation). Specifically, the present ferric hydroxide essentially contains ferric iron and has oxygen atoms or hydroxyl groups that are hexacoordinated to iron atoms so that the hexacoordinated irons are presumably linked via oxygen atoms. It is then presumed that certain water molecules present around such iron atoms will have some influence on the bond between the iron atoms and the oxygen atoms, consequently destabilizing the bond. It is then believed that, as a result of replacement of the hydroxyl groups or the destabilized oxygen atoms coordinated to the iron atoms with anions (phosphate ions, for example) the iron atoms will bond with those anions (phosphate ions). Under such assumption, a preferred formation is one in which -Fe-O-Fe-O-Fe- (cluster) has a moderate size due to the presence of moderate hydroxyl groups.

In a process for producing the present phosphorus adsorbent, ferrous iron is reacted with an oxidizing agent (sodium hypochlorite, for example) to obtain ferric hydroxide as described below. The reaction scheme for such a redox reaction is shown below, wherein ferric hydroxide is abbreviated as "$Fe(OH)_3$" for ease of understanding.

Formula 1 $\qquad$ $2Fe^{++} + NaClO + 5H_2O \rightarrow 2Fe(OH)_3 + NaCl + 4H^+$

As shown above, one mole of hypochlorite reacts with two moles of ferrous iron (in other words, one mole of hypochlorite is equivalent in amount to two moles of ferrous iron). In the process for production, such conditions are then established that the ferrous iron may not completely be oxidized into the ferric iron by reducing the amount of the oxidizing agent to less than the equivalent amount of the ferrous iron (to less than one mole of hypochlorite, in the case of two moles of ferrous iron, for example) as described below.

The term such as "amorphous" or "extremely high in amorphousness" means that X-ray powder diffraction using $K\alpha$ ray of Cu as an X-ray source shows at least one non-crystalline halo pattern in the range of 5° to 80° by $2\theta$ value, with no apparent crystalline peaks. Slight crystalline peaks may be observed in non-crystalline halo patterns depending on starting materials or the like during production. In such cases, the crystalline peak intensities observed in the range of 5° to 80° by $2\theta$ value in X-ray powder diffraction using $K\alpha$ ray of Cu as an X-ray source may be allowed if they are at or below 5% in relation to crystalline peaks for a corresponding crystalline reference material (% X-ray diffraction intensity/reference material). Specific % X-ray diffraction intensity/reference materials which may be used include those given by the formula below in accordance with ASTM (American Society for Testing and Materials) D3906. The number of crystalline peaks used for calculation of integrated reflection intensities is not particularly limited, but is preferably in the range of 1 to 8.

```
Formula 2


      (% X-ray diffraction intensity/reference material) =
{(Sₓ)/(S_R)} × 100
```

wherein $S_X$ represents an integrated reflection intensity of a sample, and
$S_R$ represents an integrated reflection intensity of a reference material.

Thus, although the active ingredient is ferric hydroxide, ferrous species are inevitably contained since the production is performed under such conditions that ferrous species (ferrous hydroxide, for example) are present. The content of such ferrous species (ferrous hydroxide, for example) is not particularly limited and usually 5% by weight or less, preferably from 0.01 to 4% by weight and more preferably from 0.1 to 2% by weight on the basis of dry weight (furnace-dried at 105°C for 2 h). The ferrous species are inevitably contained during production and such ingredients may, however, be removed by washing.

Further, the present phosphorus adsorbent may contain crystalline ferric hydroxide as long as amorphous ferric hydroxide as the active ingredient is present. In such cases, the amorphous ingredient comprises preferably 30% or more, more preferably 50% or more and even more preferably 75% or more.

It is preferred that the present phosphorus adsorbent further contains glycerin. Depending on the method of drying or aging (extended storage), the ferric hydroxide causes its OH groups attached to irons of -Fe-O-Fe-O-Fe- to be dehydrated and assumes a stable condition through growth of clusters or otherwise, possibly decreasing its adsorptive power. Therefore, admixing glycerin to wetted ferric hydroxide makes it difficult for dehydration of OH groups to occur even when it gets dry so that a decrease in adsorptive power may remarkably be inhibited. The content of glycerin is preferably 20% by weight or less on the basis of dry weight (furnace-dried at 105°C for 2 h).

A process for producing the phosphorus adsorbent according to the best mode will then be described. The present phosphorus adsorbent is obtained by (Step 1A) adding to an aqueous solution of ferrous iron an oxidizing agent (an aqueous hypochlorite solution, for example) in an amount less than the equivalent amount of the ferrous iron (preferably from 0.3 to 0.95 and more preferably from 0.4 to 0.8) or (Step 1B) adding an oxidizing agent (an aqueous hypochlorite solution, for example) to an aqueous solution of ferrous iron in such a manner that its redox potential may be brought in the range of +400 to 770 mV (preferably in the range of +500 to 730 mV and more preferably in the range of +600 to 700 mV) and then (Step 2) adding an alkali (preferably a caustic alkali) in such a manner that its pH may be adjusted in the range of 1.5 to 5.5 (preferably in the range of 1.5 to 4.0 and more preferably in the range of 2.0 to 3.5). The order of Step 1A or Step 1B and Step 2 is important. If reversed, phosphorus adsorbents having high adsorptive power could not be obtained. Each condition will be described below.

First, ferrous salts which may be used in the aqueous solution of ferrous iron are not particularly limited as long as they are water-soluble, examples of which may include ferrous sulfate, ferrous chloride and ferrous nitrate. Ferrous sulfate is preferred because filtration of precipitate is easy. Further, the concentration of ferrous ions in the aqueous solution of ferrous iron is preferably from 0.05 to 2M.

Next, oxidizing agents which may be used are not particularly limited and are preferably hypochlorites. Examples of hypochlorites may include sodium hypochlorite and calcium hypochlorite, sodium hypochlorite being particularly preferred. The concentration of a hypochlorite in an aqueous hypochlorite solution is not particularly limited and commercially available solutions with concentrations of 5 to 10 % are usable.

When Step 1A is adopted, the amount of an oxidizing agent is to be less than the equivalent amount of ferrous iron in an aqueous solution of ferrous iron. The amount of the oxidizing agent is preferably from 0.3 to 0.95 and more preferably from 0.4 to 0.8 in equivalence ratio relative to the amount of the ferrous iron.

Also, when Step 1B is adopted, an oxidizing agent (an aqueous hypochlorite solution, for example) is added to an aqueous solution of ferrous iron in such a manner that its redox potential may be brought in the range of +400 to 770 mV (preferably in the range of +500 to 730 mV and more preferably in the range of +600 to 700 mV). It is preferred that the addition of a solution of oxidizing agent (an aqueous hypochlorite solution, for example) is made dropwise while stirring.

Steps 1A and 1B may not necessarily be independent steps, so that embodiments in which performing Step 1A results in performing Step 1B and vice versa may be included.

Next, after adding a predetermined amount of oxidizing agent in Step 1A or after confirming that the redox potential has settled within the specified range in Step 1B, Step 2 of adding an alkali will be performed. Alkalis are not particularly limited and preferably are caustic alkalis. Examples of caustic alkalis may include caustic soda and caustic potassium, caustic soda being preferred. In addition, the concentration of an alkali (preferably, the concentration of a caustic alkali) is from 0.5 to 5 N, for example. Then, to the solution to which the predetermined amount of oxidizing agent has been added (Step 1A) or the solution whose redox potential has settled within the specified range (Step 1B) an aqueous alkali solution (preferably, an aqueous caustic alkali solution) is added in such a manner that its pH may be brought in the range of 1.5 to 5.5 (preferably in the range of 1.5 to 4.0 and more preferably in the range of 2.0 to 3.5). Through this procedure, amorphous ferric hydroxide will precipitate, providing the present phosphorus adsorbent.

The present phosphorus adsorbent is preferably in dry form for the ease of handling. A method for drying is preferably dehydration, freeze-drying or spray-drying, through which dehydration from the Fe-OH bond is reduced during drying so that phosphorus adsorptive power may remain high.

Further, admixing glycerin before, during or after drying can minimize a decrease in phosphorus adsorptive power. The amount of glycerin added is 20% or less (preferably from 3 to 7%) on the basis of dry weight (furnace-dried at 105°C for 2 h). The timing for admixing glycerin is not particularly limited and is preferably after pH adjustment and before drying.

Next, description will be made on uses and methods for use of the present phosphorus adsorbent. The present phosphorus adsorbent is useful in various fields where excessive phosphorus and biosafety may cause problems, such as for patients with chronic renal failures and artificial dialysis. Specific description will be made below.

First, when the present phosphorus adsorbent is used as an agent for preventing, improving or treating phosphorus-related disorders (phosphorus absorption inhibitor) for patients with chronic renal failures and artificial dialysis, it is believed optimum that the agent is filled in an enteric capsule to be orally administered. Doses are from 1 to 5 g a day depending on the conditions of patients, especially the severity of renal failures and the blood concentration of phosphorus. Thus, when the present ferric hydroxide is used for patients with chronic renal failures and artificial dialysis, the ferric hydroxide will bond with phosphate ions to form water-insoluble iron phosphate, which will be excreted with stool. The reaction of the ferric hydroxide with the water-soluble phosphate ions will complete within one minute. Therefore, almost 100 % of phosphate ions produced when foodstuff passes through the small intestine for two to three hours will be adsorbed by the iron hydroxide to be excreted. In addition, when the agent is orally administered in practical doses, it is unlikely that iron ions derived from the substance according to the present invention are absorbed in the digestive tract.

The present phosphorus adsorbent may be used as an oral preparation in view of preventing various diseases attributable to excessive phosphorus intake. Specifically, when the present preparation is orally taken along with foods and beverages or pharmaceuticals containing phosphorus, phosphoric acid produced in the intestines will be adsorbed by the present substance. Thus, the present preparation is extremely effective in preventing phosphorus-related disorders due to the intake of phosphates in large quantities. Doses are from 1 to 5 g a day, for example, depending on the age and weight of a person and the kind and quantity of a food or beverage to be taken or the like. The preparation may be added to foods and beverages or pharmaceuticals to be orally taken into the body (additive, for example) or may be taken in a physically separate form (supplement, for example).

EXAMPLES

Production of Phosphorus Adsorbent (Agent for Preventing, Improving or Treating Phosphorus-related Disorders, Oral Preparation)

A 6% sodium hypochlorite (active chlorine 5%) solution was added dropwise while stirring to 800 ml of a 0.1 M aqueous ferrous sulfate solution (amount added dropwise = 29.8 g, equivalent ratio = 0.588) in such a manner that its redox potential was 650 mV and the solution was left for three minutes while stirring. Next, 1N caustic soda was added to the solution until its pH was stabilized at 2.7 to obtain the phosphorus adsorbent according to the example. The pH at the end of the reaction was 2.7 and the redox potential was +584 mV.

Component Analysis

(1) Quantitative Analysis according to Fe Formation

For the phosphorus adsorbent obtained above, quantitative analysis was performed with respect to T-Fe, M-Fe, $Fe^{2+}$ and $Fe^{3+}$ ("T" means total and "M" means metal). For T-Fe, stannous chloride reduction-potassium dichromate titration was used for measurement, for M-Fe, mercuric chloride dissolution-potassium dichromate titration was used for measurement, for $Fe^{2+}$, inert gas-filled acid dissolution-potassium dichromate titration was used for measurement and, for $Fe^{3+}$, calculations were made according to the equation [$Fe^{3+}$ = T-Fe-(M-Fe+$Fe^{2+}$)]. The results are summarized in Table 1. Sample No. 1 is a paste-like phosphorus adsorbent and Sample No. 2 is a freeze-dried phosphorus adsorbent.
[Table 1]

| Quantitative Analysis Results (unit: wt%) | | | | |
|---|---|---|---|---|
| sample | T-Fe | M-Fe | $Fe^{2+}$ | $Fe^{3+}$ |
| No. 1 | 43.6 | less than 0.1 | 0.5 | 43.1 |
| No. 2 | 43.8 | less than 0.1 | 1.0 | 42.7 |

(2) Identification of Compositional Phase by X-ray Diffractometry (XRD)

Equipment: Type RINT-2200, Rigaku Corporation
Tube: Cu
Voltage-Current: 40 kV-40 mA
Scan Rate: 4°/min
Scan Range: 5° to 80° (2θ)

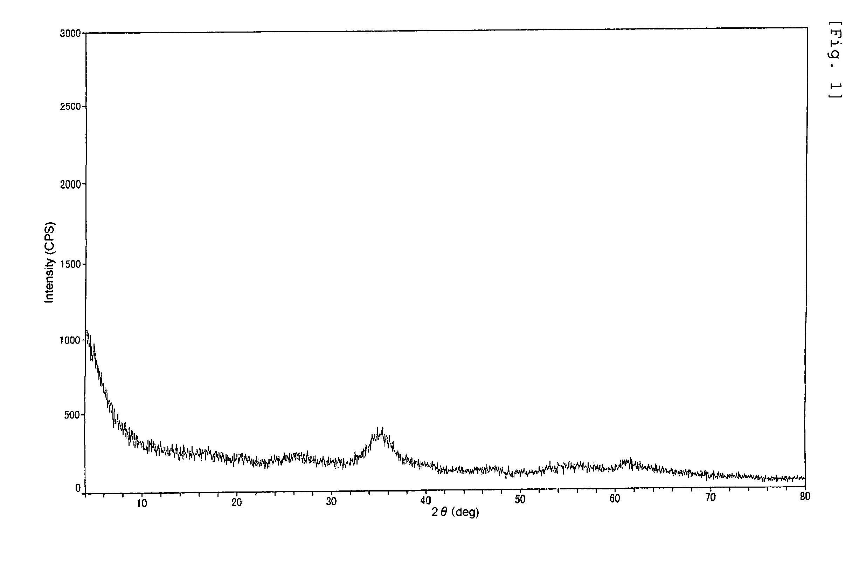

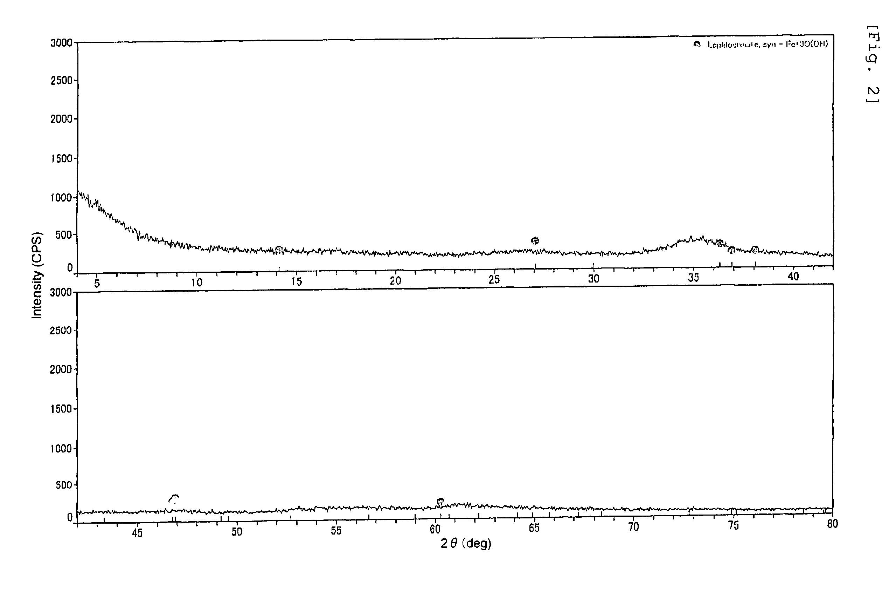

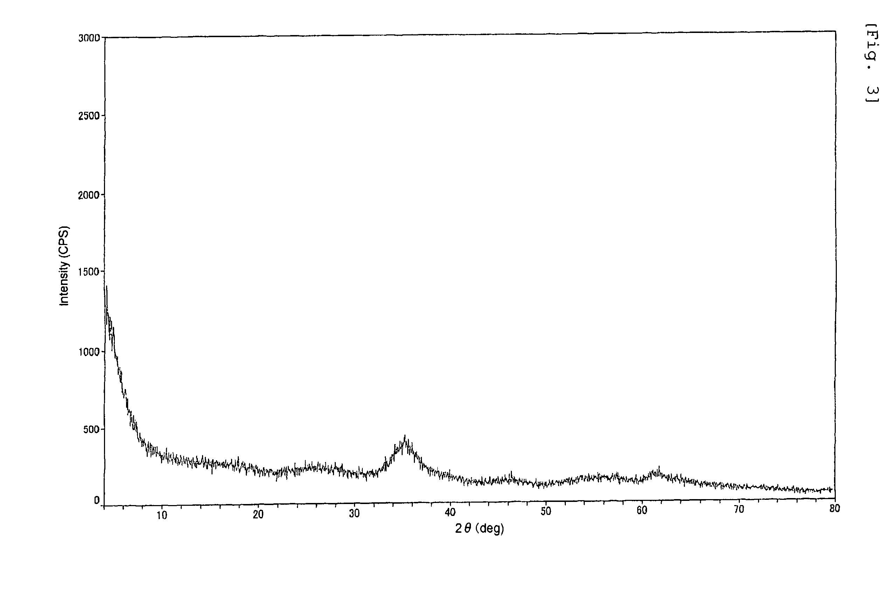

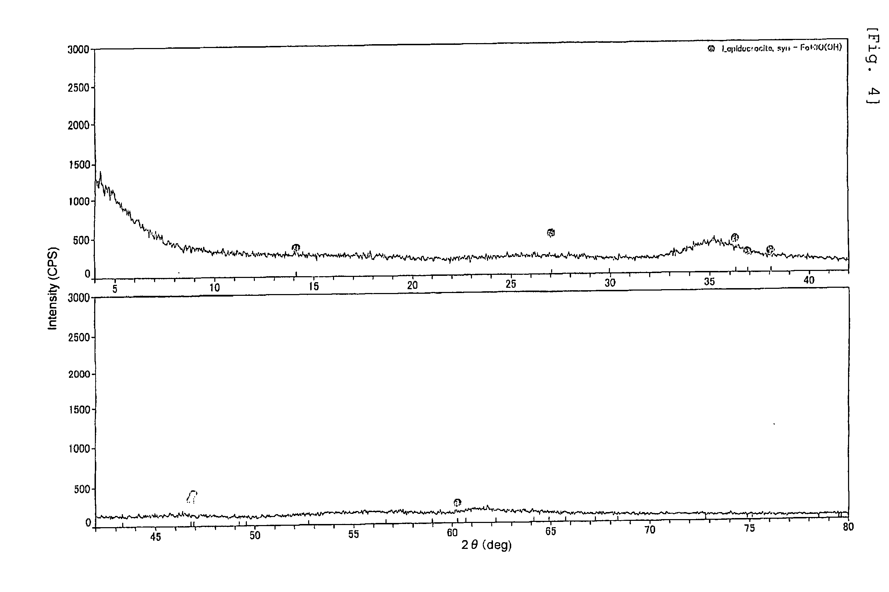

X-ray diffraction experiment was performed under the measurement conditions above. X-ray diffraction charts are shown in Figs. 1 to 4 and the results of analysis are summarized in Table 2.
[Table 2]

| X-ray Diffraction Results | | |
|---|---|---|
| compositional phase/sample name | relative intensity | |
| | No. 1 | No. 2 |
| Lepidocrocite γ-FeOOH | (+) | (+) |
| amorphous ingredient | +++ | +++ |
| relative intensity: ++++ very high, +++ high, ++ moderate, + low, (+) very low, - undetectable | | |

Based on the analysis results, lepidocrocite (γ-FeOOH) was detected as very low in intensity for both Samples No. 1 and No. 2. Also, the both samples showed generally broadened profiles except the diffraction peaks obtained in the X-ray diffraction charts, eliciting extremely high amorphousness.

Phosphorus Adsorption Test

For determining phosphorus absorptive power, 20 ml of an ammonium phosphate solution (5.9 g P/l) were added to 0.5 g (dry weight) of the phosphorus adsorbent according to the example and the solution was left for 24 hours with occasional shaking. The solution was then filtrated and the concentration of phosphorus in the filtrate was determined and calculated. For comparison, adsorptive power was also tested in a similar procedure for ferric hydroxide produced by rapidly stirring 1N NaOH into a 1M aqueous $FeCl_3$ solution in such a manner that its pH may be brought in the range of 7.5 to 8.0 and hydrous iron oxide (commercial product) produced through dehydration of ferric hydroxide. The results are summarized in Table 3.

[Table 3]

|  | Example | ferric hydroxide | commercial product (hydrous iron oxide) |
|---|---|---|---|
| phosphorus adsorptive power | 156 g/kg | 56 g/kg | 14.8 g/kg |

Example of Addition of Glycerin, etc.

Glycerin, ethanol and skimmed milk were added to the 70% hydrous phosphorus adsorbent produced according to the above procedure each at 5% by weight based on the phosphorus adsorbent and were freeze-dried. Phosphorus adsorptive power of each of these dried products is summarized in Table 4.

[Table 4]

|  | hydrous | freeze-dried | 5% glycerin added, freeze-dried | 5% EtOH added, freeze-dried | 5% skimmed milk added, freeze-dried |
|---|---|---|---|---|---|
| phosphorus adsorptive power | 156 g/kg | 102 g/kg | 130 g/kg | 117 g/kg | 123 g/kg |

Test Example 1 (Test for Confirming Action of Reducing Blood Concentration of Phosphorus Using Rats)

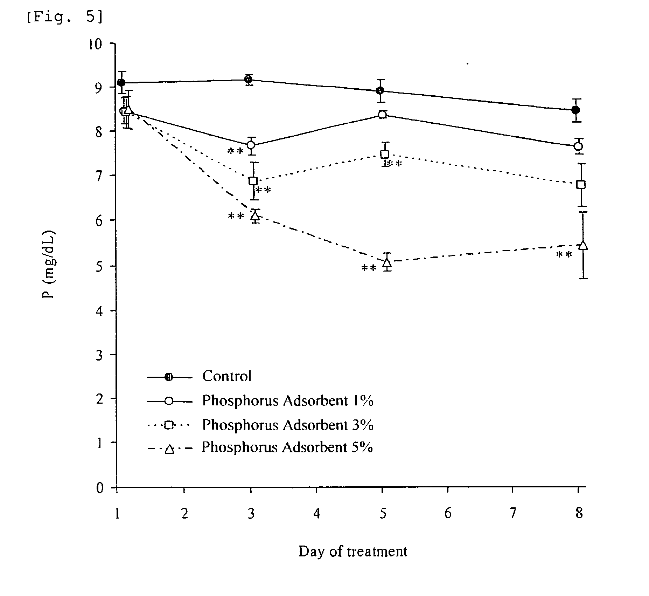

Groups each consisting of three male SD rats (eight weeks old) were bred for a week by dietary administration of a feed to which the present phosphorus adsorbent (agent for preventing, improving or treating phosphorus-related disorders) was added. Blood was sampled before the administration (Day 1), two days after the administration (Day 3), four days after the administration (Day 5) and seven days after the administration (Day 8) to determine blood concentrations of phosphorus. The group with a feed not containing the present phosphorus adsorbent was designated as Control and the groups with feeds containing 1%, 3% and 5% of the present phosphorus adsorbent were designated respectively as Phosphorus Adsorbent 1%, Phosphorus Adsorbent 3% and Phosphorus Adsorbent 5% in the drawing (Fig. 5). Also, a significant difference in phosphorus concentration from Control at each time point was indicated by ** ($P < 0.01$).

Test Example 2 (Small Intestine Fluid and Phosphorus Adsorption Test in Small Intestine System)

In order to simulate the composition of the enteral fluid with the composition of a test solution, 187 g/700 ml of an aqueous solution of an enteral nutrition Elental (AJINOMOTO PHARMA Co., Ltd.) was mixed with 1 L of Ringer solution adjusted to pH 7.2 as a small intestine fluid model to provide a test solution. To 100 ml of the test solution, 0.178 g (dry weight) of the phosphorus adsorbent (oral preparation) of the example was added and the concentration of water-soluble P in the system was determined. Also, for comparison, similar determinations were made for cases without the phosphorus adsorbent (Tests 1 and 2). Further, similar determinations were made for cases where water-soluble phosphoric acid was added (in the amount based on the assumption that the total amount of organic P in the enteric nutrition was digested in the intestines) to the test solution (Tests 3 and 4). The results are shown in the table below. As can be seen from the table, the water-soluble P was eliminated by the present phosphorus adsorbent (oral preparation) and, when a large quantity of additional water-soluble P was added, the amount of phosphorus eliminated by the present phosphorus adsorbent (oral preparation) increased. In other words, the lower the water-soluble phosphorus concentration, the less the amount adsorbed was and the higher the water-soluble phosphorus concentration, the more the amount adsorbed was. The "total P" in the table represents a total value of water-soluble and water-insoluble phosphorus. Also, the "water-insoluble phosphorus" refers to phosphorus that is not water-soluble, such as that found in proteins.

[Table 5]

| | | added water-soluble P, ppm | water-soluble P, ppm | amount of P eliminated by P adsorbent | total P, ppm |
|---|---|---|---|---|---|
| 1 | test solution | - | 29.8 | - | 155.6 |
| 2 | test solution + P adsorbent | - | 21.1 | 8.7 | 155.6 |
| 3 | test solution + added water-soluble P | 155.6 | 185.4 | - | 311.2 |
| 4 | test solution + added water-soluble P + P adsorbent | 155.6 | 83.7 | 102.7 | 311.2 |

BRIEF DESCRIPTION OF THE DRAWINGS

Fig. 1 is a chart illustrating X-ray diffraction for Sample No. 1;
Fig. 2 is a chart illustrating X-ray diffraction for Sample No. 1;
Fig. 3 is a chart illustrating X-ray diffraction for Sample No. 2;
Fig. 4 is a chart illustrating X-ray diffraction for Sample No. 2; and
Fig. 5 shows the results of Test Example 1 (action by phosphorus adsorbent of reducing blood concentration of phosphorus using rats).

**Claims**

1. An agent for preventing, improving or treating a phosphorus-related disorder comprising ferric hydroxide, wherein the ferric hydroxide is produced by adding to an aqueous solution of ferrous iron an oxidizing agent in an amount less than the equivalent amount of the ferrous iron and then adding an alkali in such a manner that its pH at the end of the reaction may be adjusted in the range of 1.5 to 5.5.

2. An agent for preventing, improving or treating a phosphorus-related disorder comprising ferric hydroxide, wherein the ferric hydroxide is produced by adding an oxidizing agent to an aqueous solution of ferrous iron in such a manner that its redox potential may be brought in the range of +400 to 770 mV and then adding an alkali in such a manner that its pH at the end of the reaction may be adjusted in the range of 1.5 to 5.5.

3. An agent for preventing, improving or treating a phosphorus-related disorder comprising ferric hydroxide, wherein the ferric hydroxide is produced by adding to an aqueous solution of ferrous iron an oxidizing agent in an amount less than the equivalent amount of the ferrous iron in such a manner that its redox potential may be brought in the range of +400 to 770 mV and then adding an alkali in such a manner that its pH at the end of the reaction may be adjusted in the range of 1.5 to 5.5.

4. The agent for preventing, improving or treating a phosphorus-related disorder according to any one of Claims 1 to 3, wherein the oxidizing agent is a hypochlorite.

5. The agent for preventing, improving or treating a phosphorus-related disorder according to any one of Claims 1 to 4, wherein the ferric hydroxide is amorphous.

6. The agent for preventing, improving or treating a phosphorus-related disorder according to any one of Claims 1 to 5, further including glycerin.

7. The agent for preventing, improving or treating a phosphorus-related disorder according to any one of Claims 1 to 6, wherein the disorder is hyperphosphatemia.

8. A process for producing an agent for preventing, improving or treating a phosphorus-related disorder, the agent containing ferric hydroxide, which comprises the steps of adding to an aqueous solution of ferrous iron an oxidizing agent in an amount less than the equivalent amount of the ferrous iron and then adding an alkali in such a manner that its pH may be adjusted in the range of 1.5 to 5.5.

9. A process for producing an agent for preventing, improving or treating a phosphorus-related disorder, the agent containing ferric hydroxide, which comprises the steps of adding an oxidizing agent to an aqueous solution of ferrous iron in such a manner that its redox potential may be brought in the range of +400 to 770 mV and then adding an alkali in such a manner that its pH may be adjusted in the range of 1.5 to 5.5.

10. A process for producing an agent for preventing, improving or treating a phosphorus-related disorder, the agent containing ferric hydroxide, which comprises the steps of adding to an aqueous solution of ferrous iron an oxidizing agent in an amount less than the equivalent amount of the ferrous iron in such a manner that its redox potential may be brought in the range of +400 to 770 mV and then adding an alkali in such a manner that its pH may be adjusted in the range of 1.5 to 5.5.

11. The process according to any one of Claims 8 to 10, wherein the oxidizing agent is a hypochlorite.

12. The process according to any one of Claims 8 to 11, wherein the ferric hydroxide is amorphous.

13. The process according to any one of Claims 8 to 12, further including the step of adding glycerin.

14. The process according to any one of Claims 8 to 13, further including the step of dehydration, freeze-drying or spray-drying.

15. The process according to Claim 14, wherein, after the step for pH adjustment, the step of adding glycerin is performed before, during or after the step of dehydration, freeze-drying or spray-drying.

16. The process according to any one of Claims 8 to 15, wherein the disorder is hyperphosphatemia.

**Patentansprüche**

1. Mittel zur Vorbeugung, Linderung oder Behandlung einer Phosphor-bedingten Störung, umfassend Eisen(III)-Hydroxid, wobei das Eisen(III)-Hydroxid hergestellt wird durch Zugabe eines Oxidationsmittels zu einer wässrigen Eisen(II)-Lösung in einer Menge, die weniger als die äquivalente Menge an zweiwertigem Eisen ist, und anschließende Zugabe einer Lauge auf eine solche Weise, dass der pH-Wert am Ende der Reaktion im Bereich von 1,5 bis 5,5 eingestellt ist.

2. Mittel zur Vorbeugung, Linderung oder Behandlung einer Phosphor-bedingten Störung, umfassend Eisen(III)-Hydroxid, wobei das Eisen(III)-Hydroxid hergestellt wird durch Zugabe eines Oxidationsmittels zu einer wässrigen Eisen(II)-Lösung auf eine solche Weise, dass das Redoxpotenzial in den Bereich von +400 bis 770 mV gebracht wird, und anschließende Zugabe einer Lauge auf eine solche Weise, dass der pH-Wert am Ende der Reaktion im Bereich von 1,5 bis 5,5 eingestellt ist.

3. Mittel zur Vorbeugung, Linderung oder Behandlung einer Phosphor-bedingten Störung, umfassend Eisen(III)-Hydroxid, wobei das Eisen(III)-Hydroxid hergestellt wird durch Zugabe eines Oxidationsmittels zu einer wässrigen Eisen(II)-Lösung in einer Menge, die weniger als die äquivalente Menge an zweiwertigem Eisen ist, auf eine solche Weise, dass das Redoxpotenzial in den Bereich von +400 bis 770 mV gebracht wird, und anschließende Zugabe einer Lauge auf eine solche Weise, dass der pH-Wert am Ende der Reaktion im Bereich von 1,5 bis 5,5 eingestellt ist.

**4.** Mittel zur Vorbeugung, Linderung oder Behandlung einer Phosphor-bedingten Störung, nach einem der Ansprüche 1 bis 3, wobei es sich bei dem Oxidationsmittel um ein Hypochlorit handelt.

**5.** Mittel zur Vorbeugung, Linderung oder Behandlung einer Phosphor-bedingten Störung, nach einem der Ansprüche 1 bis 4, wobei das Eisen(III)-Hydroxid amorph ist.

**6.** Mittel zur Vorbeugung, Linderung oder Behandlung einer Phosphor-bedingten Störung, nach einem der Ansprüche 1 bis 5, das darüber hinaus Glycerin enthält.

**7.** Mittel zur Vorbeugung, Linderung oder Behandlung einer Phosphor-bedingten Störung, nach einem der Ansprüche 1 bis 6, wobei es sich bei der Störung um Hyperphosphatämie handelt.

**8.** Verfahren zur Herstellung eines Mittels zur Vorbeugung, Linderung oder Behandlung einer Phosphor-bedingten Störung, wobei das Mittel Eisen(III)-Hydroxid enthält, umfassend die Schritte der Zugabe eines Oxidationsmittels zu einer wässrigen Eisen(II)-Lösung in einer Menge, die weniger als die äquivalente Menge an zweiwertigem Eisen ist, und anschließende Zugabe einer Lauge auf eine solche Weise, dass der pH-Wert im Bereich von 1,5 bis 5,5 eingestellt ist.

**9.** Verfahren zur Herstellung eines Mittels zur Vorbeugung, Linderung oder Behandlung einer Phosphor-bedingten Störung, wobei das Mittel Eisen(III)-Hydroxid enthält, umfassend die Schritte der Zugabe eines Oxidationsmittels zu einer wässrigen Eisen(II)-Lösung auf eine solche Weise, dass das Redoxpotenzial in den Bereich von +400 bis 770 mV gebracht wird, und anschließende Zugabe einer Lauge auf eine solche Weise, dass der pH-Wert im Bereich von 1,5 bis 5,5 eingestellt ist.

**10.** Verfahren zur Herstellung eines Mittels zur Vorbeugung, Linderung oder Behandlung einer Phosphor-bedingten Störung, wobei das Mittel Eisen(III)-Hydroxid enthält, umfassend die Schritte der Zugabe eines Oxidationsmittels zu einer wässrigen Eisen(II)-Lösung in einer Menge, die weniger als die äquivalente Menge an zweiwertigem Eisen ist, auf eine solche Weise, dass das Redoxpotenzial in den Bereich von +400 bis 770 mV gebracht wird, und anschließende Zugabe einer Lauge auf eine solche Weise, dass der pH-Wert im Bereich von 1,5 bis 5,5 eingestellt ist.

**11.** Verfahren nach einem der Ansprüche 8 bis 10, wobei es sich bei dem Oxidationsmittel um ein Hypochlorit handelt.

**12.** Verfahren nach einem der Ansprüche 8 bis 11, wobei das Eisen(III)-Hydroxid amorph ist.

**13.** Verfahren nach einem der Ansprüche 8 bis 12, das darüber hinaus den Schritt der Zugabe von Glycerin beinhaltet.

**14.** Verfahren nach einem der Ansprüche 8 bis 13, das darüber hinaus den Schritt der Dehydratisierung, Gefriertrocknung oder Sprühtrocknung beinhaltet.

**15.** Verfahren nach Anspruch 14, wobei nach dem Schritt der Einstellung des pH-Werts der Schritt der Zugabe von Glycerin vor, während oder nach dem Schritt der Dehydratisierung, Gefriertrocknung oder Sprühtrocknung durchgeführt wird.

**16.** Verfahren nach einem der Ansprüche 8 bis 15, wobei es sich bei der Störung um Hyperphosphatämie handelt.

**Revendications**

**1.** Agent pour la prévention, l'amélioration ou le traitement d'un trouble lié au phosphore comprenant de l'hydroxyde ferrique, dans lequel l'hydroxyde ferrique est produit en ajoutant à une solution aqueuse de fer ferreux un agent d'oxydation en une quantité inférieure à la quantité équivalente du fer ferreux et puis en ajoutant un alcali dans une manière telle que son pH à la fin de la réaction puisse être ajusté dans la plage de 1,5 à 5,5.

**2.** Agent pour la prévention, l'amélioration ou le traitement d'un trouble lié au phosphore comprenant de l'hydroxyde ferrique, dans lequel l'hydroxyde ferrique est produit en ajoutant un agent d'oxydation à une solution aqueuse de fer ferreux dans une manière telle que son potentiel redox puisse être porté dans la plage de +400 à 770 mV et puis en ajoutant un alcali dans une manière telle que son pH à la fin de la réaction puisse être ajusté dans la plage de 1,5 à 5,5.

13

**3.** Agent pour la prévention, l'amélioration ou le traitement d'un trouble lié au phosphore comprenant de l'hydroxyde ferrique, dans lequel l'hydroxyde ferrique est produit en ajoutant à une solution aqueuse de fer ferreux un agent d'oxydation en une quantité inférieure à la quantité équivalente du fer ferreux dans une manière telle que son potentiel redox puisse être porté dans la plage de +400 à 770 mV et puis en ajoutant un alcali dans une manière telle que son pH à la fin de la réaction puisse être ajusté dans la plage de 1,5 à 5,5.

**4.** Agent pour la prévention, l'amélioration ou le traitement d'un trouble lié au phosphore selon l'une quelconque des revendications 1 à 3, dans lequel l'agent d'oxydation est un hypochlorite.

**5.** Agent pour la prévention, l'amélioration ou le traitement d'un trouble lié au phosphore selon l'une quelconque des revendications 1 à 4, dans lequel l'hydroxyde ferrique est amorphe.

**6.** Agent pour la prévention, l'amélioration ou le traitement d'un trouble lié au phosphore selon l'une quelconque des revendications 1 à 5, comprenant en outre de la glycérine.

**7.** Agent pour la prévention, l'amélioration ou le traitement d'un trouble lié au phosphore selon l'une quelconque des revendications 1 à 6, dans lequel le trouble est l'hyperphosphatémie.

**8.** Procédé pour produire un agent pour la prévention, l'amélioration ou le traitement d'un trouble lié au phosphore, l'agent contenant de l'hydroxyde ferrique, qui comprend les étapes d'ajout à une solution aqueuse de fer ferreux d'un agent d'oxydation en une quantité inférieure à la quantité équivalente du fer ferreux et puis d'ajout d'un alcali dans une manière telle que son pH puisse être ajusté dans la plage de 1,5 à 5,5.

**9.** Procédé pour produire un agent pour la prévention, l'amélioration ou le traitement d'un trouble lié au phosphore, l'agent contenant de l'hydroxyde ferrique, qui comprend les étapes d'ajout d'un agent d'oxydation à une solution aqueuse de fer ferreux dans une manière telle que son potentiel redox puisse être porté dans la plage de +400 à 770 mV et puis d'ajout d'un alcali dans une manière telle que son pH puisse être ajusté dans la plage de 1,5 à 5,5.

**10.** Procédé pour produire un agent pour la prévention, l'amélioration ou le traitement d'un trouble lié au phosphore, l'agent contenant de l'hydroxyde ferrique, qui comprend les étapes d'ajout à une solution aqueuse de fer ferreux d'un agent d'oxydation en une quantité inférieure à la quantité équivalente du fer ferreux dans une manière telle que son potentiel redox puisse être porté dans la plage de +400 à 770 mV et puis d'ajout d'un alcali dans une manière telle que son pH puisse être ajusté dans la plage de 1,5 à 5,5.

**11.** Procédé selon l'une quelconque des revendications 8 à 10, dans lequel l'agent d'oxydation est un hypochlorite.

**12.** Procédé selon l'une quelconque des revendications 8 à 11, dans lequel l'hydroxyde ferrique est amorphe.

**13.** Procédé selon l'une quelconque des revendications 8 à 12, incluant en outre l'étape d'ajout de glycérine.

**14.** Procédé selon l'une quelconque des revendications 8 à 13, incluant en outre l'étape de déshydratation, de lyophilisation ou de séchage par pulvérisation.

**15.** Procédé selon la revendication 14, dans lequel, après l'étape d'ajustement du pH, l'étape consistant à ajouter de la glycérine est réalisée avant, pendant ou après l'étape de déshydratation, de lyophilisation ou de séchage par pulvérisation.

**16.** Procédé selon l'une quelconque des revendications 8 à 15, dans lequel le trouble est l'hyperphosphatémie.

[Fig. 1]

[Fig. 2]

[Fig. 3]

EP 2 044 946 B1

[Fig. 4]

[Fig. 5]

**EP 2 044 946 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2077266 A
- JP 3182259 A
- JP 7002903 A
- WO 0166607 A